# EUROPEAN PATENT SPECIFICATION

(11) **EP 3 213 706 B1**
(45) Date of publication and mention of the grant of the patent: **30.01.2019**
(21) Application number: 17158974.0
(22) Date of filing: 02.03.2017
(51) Int. Cl.: A61B 17/80

(54) **PLATE FOR FIXING MANDIBULAR CONDYLAR FRACTURE DEDICATED TO PATIENT'S SIDE**
PLATTE ZUR FIXIERUNG EINER MANDIBULÄREN KONDYLENFRAKTUR AUF PATIENTENSEITE
PLAQUE POUR FIXER UNE FRACTURE CONDYLIENNE MANDIBULAIRE DÉDIÉ AU CÔTÉ DU PATIENT

(30) Priority: 03.03.2016 PL 41638616
(43) Date of publication of application: 06.09.2017
(73) Proprietor: Uniwersytet Medyczny W Lodzi, 90-419 Lodz (PL)
(72) Inventor: KOZAKIEWICZ, Marcin, 92-447 Lódz (PL); KOZAKIEWICZ, Magda, 91-849 Lodz (PL)
(74) Representative: Kaminski, Piotr

(56) References cited:
- WO-A1-2004/045433

## Description

The present invention relates to a plate, dedicated to the side of the patient, for fixing of fractures of mandibular condylar fracture at each level.

A frequent consequence of injuries is a mandibular condylar fracture, which results in difficulties with food intake and speech, and facial deformity. Functioning of temporomandibular joint is not possible and occlusion disorders occur. In order to restore efficiency of the joint bone segments should be set and fixed as soon as possible, and moreover, such fixation should have appropriate strength, as at the time of biting strong forces act in this region of mandible in different directions. Dedicated plates for fixing of condylar process fractures in the shape of a delta, triangle, rhombus, letter A, Y and letter lambda are known, as described in WO 2004/045433. Some of them are universal (delta, triangular, rhombic-shaped, Y-shaped, XCP plates), and some are dedicated to the particular side of patient's body (lambda plate and A-shaped plate). Some of the plates have small dimensions (triangular plates, rhombic-shaped plates and most of delta-shaped plates) which allows for their use with an endoscope assistance. Other plates are relatively large (lambda plate, A, Y-shaped plates, some of delta-shaped plates) and intended for use by conventional skin approach. The advantage of all these dedicated plates is accordance to the lines of compression and stretching of the mandibular condylar bone in the structure, which occur during the act of mastication. This ensures a stable osteosynthesis of fractures and protects against plate breakage during the healing period. Another important design procedure increasing the breaking strength is thickening the bridges present in the lambda plate, triangular plate, and A plate and XCP.

From the clinical point of view it is important for the upper part of the plate to be narrow, so that it could be screwed into the narrow bone of the neck of the mandible. It is well presented by lambda plate. It was observed that the stability of the fracture fixing depends on the number of fastening screws, in particular in the upper segment. Most dedicated plates have only the possibility of mounting two screws in the upper segment. Stability is much higher when three screws are used, which were applied in plate A and XCP.

By way of example, A-shaped plate is discussed in detail in the publication of the Polish patent application no. P.400191. The plate presented therein has two arms connected together at the top and parted at the bottom and there connected with a crossbar, wherein the screw holes for fixing the plate in the condyle are at the top at the arm joint and, in the bottom of each arm there are holes for screws for fixing the plate in the ramus of the mandible.

The XCP plate is a plate in the shape of letter A comprising two arms connected together at the top and parted at the bottom and there connected with a crossbar, wherein, three holes for screws for fixing the plate in the condyle are located at the joint of said arms, said holes being arranged at the top of the vertices of an isosceles triangle, and at the lower ends of the arms there are two holes for screws for fixing the plate in the ramus of the mandible. The plate is symmetrical relative to axis of symmetry and the crossbar is an X-shaped crossbar with the axis of symmetry coinciding with the axis of symmetry of the plate, wherein four arms of the crossbar are directed towards the respective holes for screws for fixing the plate in the ramus of the mandible.

A disadvantage of the A-shaped plate is that it has the lower ends which require to set up as many as 6 screws, wherein these ends are quite long and transversely not stabilised. On the other hand, a disadvantage of the universal XCP plate is incomplete accordance to the lines of compression and stretching of the mandibular condylar bone in the structure coincidence with compression and tension lines in case of low subcondylar fractures. It causes that in the case of use of XCP plate for low subcondylar fractures, the plate should be larger. Additional attachment sites are also worth considering.

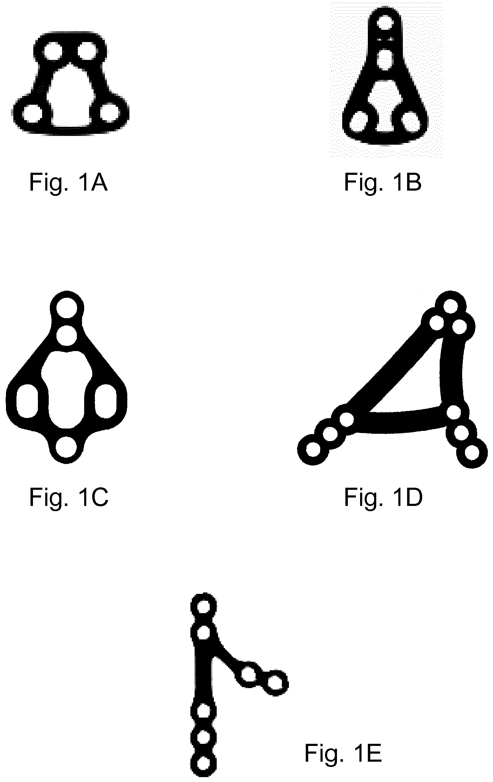

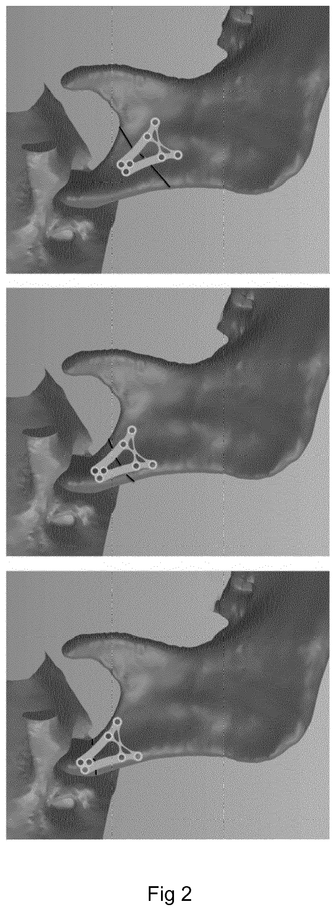

Examples of respective types of the above discussed plates are presented in Fig. 1A-1E, where Fig. 1A shows an example of delta plate, Fig. 1B shows an example of triangle-shaped plate, Fig. 1C shows an example of rhombus-shaped plate, Fig. 1D shows an example of A-shaped plate, Fig. 1E shows an example of lambda plate. Fig. 2 shows XCP plate on the ramus of the mandible, fixing mandibular condylar fractures at different levels.

It would be advisable to develop a new plate design that would be characterised by high strength and which could be easily used for fixing of low subcondylar fractures.

The subject matter of the invention is a plate dedicated to the side of the patient for fixing of fractures of mandibular condylar fracture, in the shape of letter A comprising two arms connected together at the top and parted at the bottom and there connected with a crossbar, wherein three holes for screws for fixing the plate in the condyle are located at the joint of said arms, said holes being arranged at the top of the vertices of an isosceles triangle, while at the lower ends of the arms there are two holes for screws for fixing the plate in the ramus of the mandible, which plate has a first arm and a second arm outwardly bent in relation to each other, wherein said arms are in the shape of arc, the radius of the arc of the first arm is greater than the radius of arc of the second arm, the crossbar is a cross-shaped crossbar in the shape of letter X, wherein four arms of the crossbar are directed towards the respective holes for screws for fixing the plate in the ramus of the mandible.

Preferably, the holes for screws for fixing the plate in the condyle are arranged at the vertices of an isosceles triangle, wherein the vertex of the triangle opposite the base of the triangle is directed towards the crossbar.

Preferably, the upper part of the first arm and the upper part of the second arm between the holes for screws for attaching the plate in the condylar process and the holes for screws for attaching the plate in the ramus of mandible, and the lower part of the first arm and the lower part of the second arm between two holes for screws for attaching the plate in the ramus of mandible, have the shape of arched bridges, bent along the lines of compression and stretching of the mandibular condylar bone.

Preferably, the width of the upper parts of the first and second arms is greater than the width of the lower parts of the first and second arms.

Preferably, the holes are threaded.

The plate according to the invention is presented in the drawing, in which Fig. 3 shows the plate in a front view dedicated to the patient's right side, Fig. 4 shows the plate in a front view dedicated to the patient's left side, Fig. 5 shows the plate on the ramus of mandible fixing low subcondylar fractures.

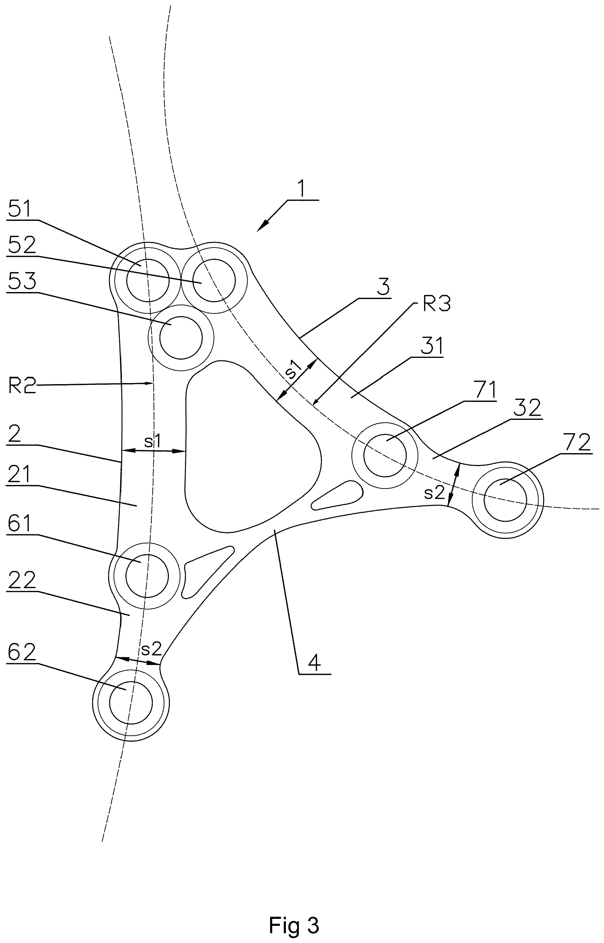

The plate 1 according to the invention, as shown in an embodiment in Fig. 3, has the shape of an asymmetrical letter A, the first arm 2 and second arm 3 of which are connected by means of a crossbar 4. In the place of connection of the first arm 2 with the second arm 3 there are three holes 51, 52, 53 designed to accommodate screws for fixing the plate 1 in the condyle. The holes 51, 52, 53 are arranged at the vertices of isosceles triangle, the vertex of which having the hole 53 and being disposed opposite to the triangle base is directed towards the upper part 21 of the first arm 2. In the lower parts of the first arm 2 and second arm 3 of the plate 1 there are, respectively, pairs of holes 61, 62 and 71, 72 designed to accommodate screws for fixing the plate 1 in the ramus of mandible, wherein one of the holes 61, 71 in each pair is located at the height of the crossbar 4, while the other one of the holes 62, 72 in each pair is located below the crossbar 4 (i.e. in the ends of the arms 2, 3).

The crossbar 4 is a crossbar in the shape of letter X, four arms of which are directed towards the particular holes 61, 62 and 71, 72 in the lower parts of the first arm 2 and second arm 3 arm.

The upper parts 21, 31 of the first arm 2 and second arm 3, between the holes 51 and 61, and 52 and 71, as well as the lower parts 22, 32 of the arms 2, 3, between the holes 61 and 62, and 71 and 72, have the shape of arched bridges, bent in parallel to the known lines of compression and stretching of the bone in the condyle and ramus of mandible during chewing act.

As shown in Fig. 3, both of the first and the second arms, have the shape of arc and are outwardly bent in relation to each other, wherein the radius R2 of arc of the first arm 2 is greater than the radius R3 of arc of the second arm 3. In the most preferred embodiment of the plate according to the invention, the radius R2 of arc of the first arm is four times greater than the radius R3 of arc of the second arm. The width s1 (measured at the narrowest place) of the upper parts 21, 31 of the first arm 2 and second arm 3 arm can be greater, in exemplary embodiment by 50%, than the width s2 (measured at the narrowest place) of the lower parts 22, 32 of the arms 2, 3.

The plate can be of small size, having for example the height of about 23 mm and the width of about 21 mm, and thickness from 1 mm to 1.3 mm. The width s1 can be 3 mm and the width s2 can be 2 mm. The holes 51, 52, 53, 61, 62, 71, 72 can be adapted to accommodate screws having the diameter of 1.8 mm to 2.0 mm and they can be threaded. The length of the radius R2 of arc of the first arm in such preferred embodiment is 80 mm, while the length of the radius R3 of arc of the second arm is 20 mm.

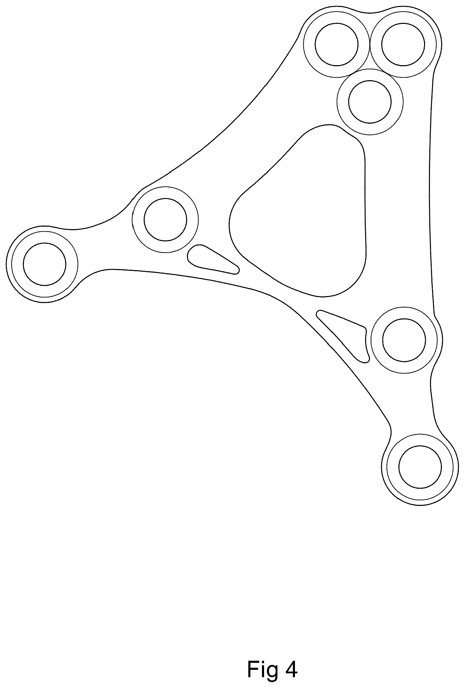

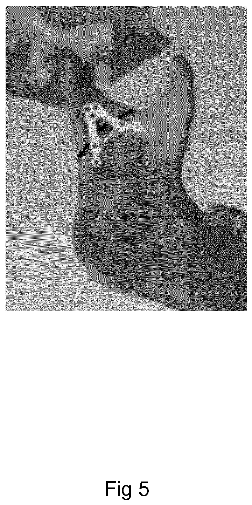

As shown in Fig. 5, the plate can be fixed on the ramus of mandible (where the fracture is marked with the solid line) at different heights but it will be the best fit for treating low subcondylar fractures. The plate is dedicated to the patient's side, i.e. one variant of the plate (Fig. 3) can be attached on the patient's right side, and the other one, being a mirror image of the first variant (Fig. 4), can be attached on the patient's left side. The use of three screw holes for fixing the plate in the condyle provides high stability of the osteosynthetised bone fragments. The design of the plate is determined by the lines of compression and stretching of the mandibular condyle, which also contributes to the stability of the mount. The use of crossbar 4 increases the rigidity of the plate and prevents from twisting of the plate at its lower part. This allows for reduction of the width of the lower parts of the arms with respect to their upper parts and allows for reduction of the number of screw holes in the lower part of the plate, which reduces the size and weight of the plate.

The plate according to the invention provides for desired mechanical features expressed by a low value of reduced stress, much lower than the yield point of medical titanium alloy.

## Claims

1. A plate dedicated to the side of the patient for fixing of fractures of mandibular condylar fracture, in the shape of letter A, comprising two arms connected together at the top and parted at the bottom and there connected with a crossbar, wherein three holes for screws for fixing the plate in the condyle are located at the joint of said arms, said holes being arranged at the top of the vertices of an isosceles triangle, while at the lower ends of the arms there are two holes for screws for fixing the plate in the ramus of the mandible, **characterised in that** the plate (1) has a first arm (2) and a second arm (3) bent outwardly in relation to each other, wherein said arms are in the shape of arc, the radius (R2) of the arc of the first arm (2) is greater than the radius (R3) of arc of the second arm (3), the crossbar (4) is a cross-shaped crossbar in the shape of letter X, wherein four arms of said crossbar (4) are directed towards the respective holes (61, 62; 71, 72) for screws for fixing the plate (1) in the ramus of the mandible.

2. The plate according to claim 1, **characterised in that** the holes (51, 52, 53) for screws for fixing the plate (1) in the condyle are arranged at the vertices of isosceles triangle, the vertex of which being opposite to the triangle base is directed towards the upper part (21) of the first arm (2).

3. The plate according to claim 1, **characterised in that** the upper part (21) of the first arm (2) and the upper part (21) of the second arm (3) between the holes respectively (51, 52) for screws for fixing the plate in the condyle and the holes for screws respectively (61, 71) for attaching the plate in the ramus of mandible, and the lower part (22) of the first arm (2) and the lower part (32) of the second arm (3) between two holes for screws respectively (61, 62; 71, 72) for attaching the plate in the ramus of mandible, have the shape of arched bridges, bent along compression and stretching lines of the bone.

4. The plate according to claim 3, **characterised in that** the width (s1) of the upper parts (21, 31) respectively of the first arm (2) and second arm (3) arm is greater than the width (s2) of the lower parts (22, 32), respectively of the first (2) and second (3) arms.

5. The plate according to claim 1, **characterised in that** the holes (51, 52, 53, 61, 62, 71, 72) are threaded.

## Patentansprüche

1. A-förmige, patientenspezifische Platte zum Verbinden von Frakturen des Unterkiefer-Gelenkfortsatzes, bestehend aus zwei oben verbundenen und unten gespreizten und unten durch eine Querstange verbundenen Schenkeln, wobei an der Verbindungstelle der Schenkel sich drei an den Ecken des gleichschenkligen Dreiecks angeordnete Schraublöcher zur Befestigung der Platte im Unterkiefer-Gelenkfortsatz und an den unteren Enden der Schenkel jeweils zwei Schraublöcher zur Befestigung der Platte im Unterkiefer-Gelenkfortsatz befinden, **gekennzeichnet dadurch, dass** die Platte (1) erste (2) und zweite (3) voneinander nach außen gebogene Schenkel aufweist, wobei die vorgenannten Schenkel die Form eines Bogens aufweisen, und der Bogenradius (R2) des ersten Schenkels (2) größer ist als der Bogenradius (R3) des zweiten Schenkels (3), und die Querstange (4) eine X-förmige Querstange ist, wobei die vier Schenkel der Querstange (4) in Richtung der einzelnen Schraublöcher (61, 62; 71, 72) zur Befestigung der Platte (1) am Unterkieferast gerichtet sind.

2. Platte nach Anspruch 1, **gekennzeichnet dadurch, dass** die Schraublöcher (51, 52, 53) zur Befestigung der Platte (1) im Unterkiefer-Gelenkfortsatz an den Ecken eines gleichschenkligen Dreiecks angeordnet sind, dessen Scheitelpunkt gegenüber der Basis des Dreiecks in Richtung des oberen Teils (21) des ersten Schenkels (2) gerichtet ist.

3. Platte nach Anspruch 1, **gekennzeichnet dadurch, dass** der obere Teil (21) des ersten Schenkels (2) und der obere Teil (22) des zweiten Schenkels (3) zwischen den Schraublöchern (51, 52) zur Befestigung der Platte im Unterkiefer-Gelenkfortsatz und den Schraublöchern (61, 71) zur Befestigung der Platte im Unterkieferast, sowie der untere Teil (22) des ersten Schenkels (2) und der untere Teil (32) des zweiten Schenkels (3) zwischen den beiden Schraublöchern (61, 62; 71, 72) zur Befestigung der Platte im Unterkieferast, jeweils die Form von Bogenbrücken haben, die entlang der Kompressions- und Dehnungslinie des Knochens gebogen sind.

4. Platte nach Anspruch 3, **gekennzeichnet dadurch, dass** die Breite (s1) der oberen Teile (21, 31) des ersten (2) und zweiten (3) Schenkels größer ist als die Breite (s2) der unteren Teile (22, 32) des ersten (2) und zweiten (3) Schenkels.

5. Platte nach Anspruch 1, **gekennzeichnet dadurch, dass** die Schraublöcher (51, 52, 53, 61, 62, 71, 72) mit einem Gewinde versehen sind.

## Revendications

1. Une plaque dédiée au côté patient pour réunir les fractures du condyle de la mandibule, en forme de la lettre A comprenant deux bras reliés l'un à l'autre en haut et évasés en bas et là, reliés par une traverse, où à l'endroit où les bras sont connectés, il y a trois trous de vis disposés aux sommets du triangle isocèle pour la fixation de la plaque dans le condyle, et à chaque extrémité inférieure des bras, il y a deux trous de vis pour la fixation de la plaque dans la branche de la mandibule, **caractérisée en ce que** la plaque (1) a les premier (2) et second (3) bras courbés vers l'extérieur l'un par rapport à l'autre, où lesdits bras ont la forme d'arc, le rayon de l'arc (R2) du premier bras (2) est plus grand que le rayon de l'arc (R3) du second bras (3), la traverse (4) est une traverse en forme de la lettre X, où les quatre bras de la traverse (4) sont dirigés vers les trous de vis respectifs (61, 62; 71, 72) pour la fixation de la plaque (1) dans la branche de la mandibule.

2. La plaque selon la revendication 1, **caractérisée en ce que** les trous de vis (51, 52, 53) pour la fixation de la plaque (1) dans le condyle sont disposés aux sommets du triangle isocèle dont le sommet opposé à la base du triangle est dirigé vers la partie supérieure (21) du premier bras (2).

3. La plaque selon la revendication 1, **caractérisée en ce que** la partie supérieure (21) du premier bras (2) et la partie supérieure (22) du second bras (3) entre les trous de vis (51, 52) respectivement pour la fixation de la plaque dans le condyle et les trous de vis (61, 71) respectivement dans la branche de la mandibule, et la partie inférieure (22) du premier bras (2) et la partie inférieure (32) du second bras (3) entre les deux trous de vis (61, 62; 71, 72) respectivement pour la fixation de la plaque dans la branche de la mandibule ont la forme de ponts en arc, courbés le long de la ligne de compression et d'étirement des os.

4. La plaque selon la revendication 3, **caractérisée en ce que** la largeur (s1) des parties supérieures (21, 31) des premier (2) et second (3) bras respectivement est supérieure à la largeur (s2) des parties inférieures (22, 32) des premier (2) et second (3) bras respectivement.

5. La plaque selon la revendication 1, **caractérisée en ce que** les trous (51, 52, 53, 61, 62, 71, 72) sont filetés.
